Europäisches Patentamt

European Patent Office

Office européen des brevets

(11) Publication number: **0 139 676 B1**

# EUROPEAN PATENT SPECIFICATION

(45) Date of publication of patent specification: **25.11.92**    (51) Int. Cl.5: **C12P 21/00**, C12P 21/08

(21) Application number: **84901131.7**

(22) Date of filing: **03.02.84**

(86) International application number:
**PCT/US84/00161**

(87) International publication number:
**WO 84/03106 (16.08.84 84/20)**

(54) **PRODUCTION AND CHARACTERIZATION OF HYBRIDOMA ANTIBODIES DIRECTED SPECIFICALLY AGAINST COMMON DETERMINANT(S) PRESENT AMONG CLOSELY RELATED, BUT DISTINCT PROTEINS.**

(30) Priority: **04.02.83 DK 457/83**

(43) Date of publication of application:
**08.05.85 Bulletin 85/19**

(45) Publication of the grant of the patent:
**25.11.92 Bulletin 92/48**

(84) Designated Contracting States:
**DE FR GB**

(56) References cited:

**Nature, 286, 12 June 1980, Secher et al., "A Monoclonal Antibody for Large-Scale Purification of Human Leukocyte Interferon" p. 446-50**

(73) Proprietor: **Cytoclonal Pharmaceutics Inc.**
**9000 Harry Hines Blvd Suite 330**
**Dallas, Texas 75235(US)**

(72) Inventor: **BERG, Kurt, Frimann**
**3817 Purdue**
**Dallas, TX 75225(US)**

(74) Representative: **UEXKÜLL & STOLBERG Patentanwälte**
**Beselerstrasse 4**
**W-2000 Hamburg 52(DE)**

EP 0 139 676 B1

EP 0 139 676 B1

Proceedings of the National Academy of Sciences USA, 78(3) March 1981, Staehelin et al., "Producation of Hybridomas Secreting Monoclonal Antibodies to the Human Leukocyte Interferons", p. 1848-52

C.R. Academy of Sciences Paris 291, 24 November 1980, Montagnier et al., "Isolement D'un Hybride Cellulaire Produisant Un Anti-Corps Specifique de L'Interferon Leucocytaire Humain", p. 893-6

Journal of Immunology, 128(6), June 1982, Imai et al., "Demonstration of Two Sub-types of Human Leukocyte Interferon (IFN-a) by Monoclonal Antibodies", p. 2824-5

Journal of Immunology 129(5), November 1982 Novick et al., "Monoclonal Antibodies to Human a-Interferon and their Use for Affinity Chromatography", p. 2244-7

Stewart et al., "Interferons and Their Actions", 1980, CRC Press, Boca Raton FL., p. 54-61

Nucleic Acids Research 8(24), December 1980, Takahashi et al., "Cloning of Human Immunoglobin u Gene and Comparison with Mouse u Gene", p. 5983-91

Nucleic Acids Research 5(6) March 1978, Hozumi et al., "Characterization of a Mouse DNA Clone Containing an Immunoglobulin Variable Region Gene", p. 1779-99 Chemical Abstracts 89: 87928t

US 4,423,147, 27 December 1983, Secher et al.

US 4,350,683, 21 September 1982, Galfre et al.

## Description

TECHNICAL FIELD

The invention is related to description, production, and characterization of new specific hybridoma/monoclonal antibodies recognizing common antigenic determinant(s) among the closely related proteins belonging to the HuIFN-a system - be they natively produced (in vivo or in vitro) or produced by recombinant DNA-techniques (genetic engeneering) or produced by peptide syntheses.

BACKGROUND ART

Interferons are now widely recognized as being an important group of biological proteins rendering cells or whole organisms resistant to virus attack (the so-called antiviral activity, cf. Stewart (1980)). Furthermore, interferons have proved to play a significant role in the regulation of the immune system in vivo and in vitro together with numerous other so-called non-viral activities, such as re-transformation of transformed cells, inhibition of growth of tumor cells, etc., cf. for example Vilceck et al. (1980). For these reasons the interferon system has during the last decade been intensively investigated and numerous findings have been reported (cf. Stewart (1980), Berg (1982)).

One of the main difficulties observed during the beginning of the interferon research was the difficulties involved in the purification and characterization of the interferon proteins. It was believed that only one protein species existed in the human system, but later, it was shown by several investigators, e.g. Berg et al. (1975), Stewart (1980), Berg (1982), that there indeed exist three main types of interferon in the human system: Human Leukocyte Interferon (HuIFN-$\alpha$), Human Fibroblast Interferon (HuIFN-$\beta$), and Human Immune Interferon (HuIFN-$\gamma$).

HuIFN-$\alpha$ has now been completely purified. Originally it was thought to consist of one single entity only, but later on, as the purification progressed, it turned out that HuIFN-$\alpha$ consists of at least 6-7 different interferon protein species, and finally it was shown that at least 13 species exist (Berg et al. (1980), (1982a, b). These species were purified simultaneously by combining traditional techniques (such as gelfiltration and the like) with antibody affinity chromatography using antibodies from rabbits. The pure specie was next, in turn, used as immunogen whereby "monospecific" rabbit antibodies against the interferon proteins were produced by using only a single interferon specie (e.g.: 20.000 specie) as immunogen. Thus, the monospecific, polyclonal rabbit antibodies were able to purify all species of HuIFN-$\alpha$ simultanously when used in an antibody affinity column. This approach was followed by others employing immunoglobulins produced from hybridomas, (e.g.: Secher et al. (1980)). However, there was a serious drawback to this method: only part of the interferon proteins was bound to the hybridoma column (cf. Berg et al (1982, b). Usually, half of the interferon activity was found in the wash, and the other half was bound to the column (and could be eluted specifically). Therefore, the impression was, generally speaking, that HuIFN-$\alpha$ might not contain a common antigenic determinant, at least not as measured by means of the mouse hybridoma system.

SUMMARY OF THE INVENTION

The present invention addresses itself to this problem. In the following it will be shown that it, now, indeed is possible to produce hybridoma-produced antibodies (immunoglobulins) which are specific for at least one antigenic determinant common to the 12 species of Human Interferon-alpha demonstrating activity in the human system and having molecular weights of at least 16000 in SDS-PAGE, a species having a molecular weight of 16600 being excluded, as described by Berg et al. (1982a) and (1982b). The invention is new and surprising compared to the previous knowledge. The implications of the present inventions are immanent:

all proteins belonging to HuIFN-$\alpha$, all the bacterial derived HuIFN-$\alpha$ species-obtained by genetic engineering techniques) will bind this immunoglobulin (which is characterized in that it binds all species of HuIFN-$\alpha$ as shown in an antibody affinity chromatography), whereby a universal approach for purifying all species of HuIFN-$\alpha$ be it native ones or bacterially (plasmid) derived - have been invented for the first time. It is also within the scope of this invention to use other hybridoma systems (apart from the murine system) yielding hybridoma antibodies (or the essential parts thereof) of the specificity as defined above. For example, it is foreseen that also the rat system will be used in the future.

The said immunoglobulins might also be produced by the now well known genetic engineering techniques, for example by means of the hybridoma cells producing the said immunoglobulins by first

3

EP 0 139 676 B1

isolating the proper m-RNA (the specificity of which can be studied/measured/isolated by very well known standard techniques as for example illustrated in Methods in Enzymology, vol. 68 (1981), Recombinant Techniques. The purified m-RNA, which can be tested for the correct specificity in oocytes (by means of a semi-solid binding assay of the translated product - vide Materials and Methods in the present application) can then be transformed to c-DNA by means of reverse RNA-transscriptase and after making a plasmid library by incorporating the said c-DNA into relevant plasmids, the correct cloned can be found by screening taking into account that a probe can be made out from the correct m-RNA (identified/isolated/described as shown above), cf. Weissmann (1982). It is also within the scope of the said invention to produce only parts of immunoglobulins showing the desired specificity.

The said immunoglobulins will be very useful in different immunological assays employing, for example ELISA-(enzyme-immuno) or RIA-(radio-immuno) techniques or the like, in which the presence of a monoclonal antibody molecule (or parts thereof), with the above specificity is essential. Thus, the said immunoglobulins may be very useful in immuno assays for detecting interferon proteins (or parts thereof) by means of conventional techniques (see example Methods in Enzymology, vol. 70, part A, 1980).

It will also be possible to detect (quantity/quality) the presence of interferon receptors (which, in advance, have received interferon proteins, or parts thereof) or the like since these entities also can be recognized by the above antibodies.

BEST MODES OF CARRYING OUT THE INVENTION

It is contemplated that one of the reasons for being successful in obtaining the said hybridoma antibodies showing the remarkable binding properties can be found in the method for preparing the antigen-preparations consisting of highly purified interferon proteins stabilized with SDS (cf. Berg et al. (1980), (1982a)). The presence of SDS is thought to be an important factor in the presentation of the proteins to the immunocompetent cells in that the proteins in question (e.g.: interferon species) are exposing all their antigenic determinants in the presence of SDS (the proteins can also be pretreated with SDS before being confronted with the immunocompetent cells), due to the denaturating power of SDS, whereby otherwise hidden determinants are fully exposed. It is also within the scope of this invention to treat proteins, in general, with SDS before (or simultaneously) the immunization is carried out the purpose of this procedure being to obtain more hidden antigenic determinants which otherwise are not recognized. By employing this "SDS-approach" during the course of immunization it is to be expected that the hybridoma antibodies against the most frequent determinants (i.e.: that or those found to be most common among all the proteins belonging to a protein mixture composed by related, but nevertheless, distinct proteins) will be found more often when using such a denaturing approach during the course of preparing the relevant immunogens (comprizing proteins, e.g.: pure species of HuIFN-$\alpha$). This general principle applies also to other hybridoma systems apart from the murine system.

The above findings correlate very well with the well known homology which has been shown to exist on the gene level, for the genes coding for HuIFN-$\alpha$, cf. Weissmann et al. (1982). Since several regions are known to be constant (cf. Weissmann et al. (1982)) it is contemplated that this hybridoma, which is defined by the mere ability to recognize all species of HuIFN-$\alpha$, probably recognizes a specific determinant located in such a region, which is common for all the interferon species belonging to the HuIFN-$\alpha$ species. It is also contemplated that the said antibody also might recognize MuIFN-$\alpha$ having the same determinant as involved in the said specificity of the said antibodies. It is not known, at present, how widely distributed this determinant might be in the interferon-system (work is in progress).

For immunization, isolation of a spleen lymphocyte mixture fusion and hybridization, growing of hybrids and selection, subcloning of hybrides producing the desired antibody and isolations of these hybrids or the corresponding antibodies, the reader is referred to numerous previously published descriptions, as f.x, Kohler et al. (1975), (1976), Lovborg (1982), Methods in Enzymology, Vol. 70 (1980), Secher et al (1980), Cellular Immunology, Vols. 1-3 (1978), Kennett, McKern & Bechtol (1980).

Most of the plasmacytoma cell lines used to make hybridomas and some hybridomas producing antibodies are available from public cell distributing centers, such as for example The Salk Institute, Cell Distribution Center, P. O. Box 1809, San Diego, Ca. 92112, USA, or other similar places. The present cell line, used in this invention, was obtained from The Human Genetic Mutant Cell Repository in New Jersey, wide the section called: cells. The cells are generally grown in Dulbecco's Modified Eagle's Medium (DMEM) with high glucose (4, 5 g pr liter) or in RPMI 1640. Addition of glucose to RPMI 1640 up to 4, 5 g pr liter does improve growth at higher densities. Cells are maintained in stationary suspension culture at a concentration of $10^5$-$10^6$ cells pr. ml. Optimum condition of a culture to be used for fusion is high viability (i.e.: greater than 90%) with 3-8 x $10^5$ cells pr. ml. For more experimental details the reader is referred to

4

f.x. Lovborg (1982) and Kennett et al (1980).

Test for Mycolplasma was always performed since it was shown by others that the presence of mycoplasma very easily can hinder a successful hybridization. All myeloma cells were always checked 1-3 days before used. Only if the test turned out to be absolutely negative were the cells accepted for fusion.

The institution of the mycoplasma test into the hybridization experiments greatly improved the numbers of positive hybridomas obtained in the fusion experiments, thus confirming similar observations performed by several groups (cf. Lovborg (1982)).

EXPERIMENTAL SECTION

Interferon titrations

Were performed as previously described using for example VERO-cells and VSV as chellenge virus (see f.x. Berg et al. (1980)). All units are expressed into international units (69/19 B, MRC, UK). If very low amounts of interferon were to be detected the so-called bovine system, comprising BMDK-cells and VSV, was used since it turned out to be extremely reliable and very sensitive. As low as 0.1 units of interferon activity could easily be detected. Therefore, this system was widely used in the semi-neutralization of hybridoma-supernatants.

Preparation of the immunogen

Crude human leukocyte interferon was purified by gelfiltration followed by an antibody affinity chromatography yielding almost pure interferon proteins with a specific activity of $10^8$ units pr. mg. protein. Recovery was about 75%. The total eluate from a typical experiment contained $5 \times 10^6$ units which was divided into 100.000 units containing aliquotes (in 0.1 ml eluting buffer containing 0,1% SDS) and kept at -20°C until used (stable for at least 5 months) - cf. Berg et al. (1980). Sometimes, less purified preparations of leukocyte interferon were also used (inc. SDS).

Immunization

The Balb/c mice-preferentially female mice - were immunized as follows: The first injection (100.000 units) was mixed with Freund's Adjuvans (F.A.) and given intra peritoneally (I.P.). The following 5-8 injections were given as weekly injections but consisted only of 30.000 units and no F.A. was included. After 5-8 injections the mice developed antibodies to HuIFN-$\alpha$ (detected by the "traditional" neutralization test (wide neutralization tests) in the range of 200 - 2000 neutralizing units/ml - cf. Berg (1982). This estimate very often yielded considerably lower figures compared to the semi-solid neutralization method (wide below) which turned out to be far more sensitive. Two - five days before the fusion the mice received 100.000 units in total as a booster: half as I.P. and the other half as subcutaneous (S.C.) injections without F.A.

NEUTRALIZATION OF ANTIBODIES TO HuIFN-$\alpha$

The simple ("traditional") method for neutralization of interferon consists of mixing equal volume of interferon proteins containing a predetermined amount of interferon units with the antibody sample in question using several dilution step, see f.x. Berg (1982). This method, however, will only recognize those antibodies reacting with the antigenic determinants close to the biological determinants on the interferon molecule(s). Thus, as pointed out by Berg (1982), it is quite possible that a great deal of antibodies will escape identification by using the "traditional" neutralization method only.

To overcome this problem several ELISA-tests were employed taking into account that f.x. sheep antibodies to HuIFN-$\alpha$ (produced out from partially purified HuIFN-$\alpha$ by ethanolic precipitation, cf. Stewart (1980)) bind all species of HuIFN-$\alpha$. Thus, immunoplates (NUNC-DENMARK) were firstly coated with IgG-solutions from a sheep antiinterferon serum followed by pure interferon proteins and after a suitable incubation the samples from the hybridomas were added and finally rabbit antimouse IgG-galatosidase conjugates were added, as detailed in details in f.x. Methods in Enzymology, vol. 70, part A, 1980). This method turned out, however, to yield too many false positive results in the sense that ELISA-positive supernatants did not turn up as positives in the semi-solid neutralization test (wide below).

SEMI-SOLID NEUTRALIZATION TEST

This method was developed in order to test ELISA-positive samples. The test takes advantage of the very simple fact that a "possible" antibody molecule directed against HuIFN-α will bind to the interferon molecule. Therefore, if one first immobilizes the "possible" antibody (which can be done by another antibody directed against the said "possible" antibody) before adding an exact, but small amount of interferon, preferably as low as 0,1 units in total (wide the section of titration of interferon) a very small drop in the interferon titer (content) could easily be detected using the "bovine" system. This system proved superior to the ELISA-system. The following method was used:

1. Coat with rabbitanti mouse immunoglobulin (DAKO-PATT, Denmark) using 300 pg/ml in PBS, inc. 0,05% sodium azide (S.A.) at 37°C for 1 hour. Transfer to 4°C for at least 24 hours (or several weeks).

2. Wash with PBS incl. 0,05% S.A. incl Tween 80(0,05%) - the so-called: washing buffer - three times.

3. Saturate unbound sites of the immunoplates with 2% egg albumin in PBS (SIGMA) at room temperature for 2 hours.

4. Wash 3 times with washing buffer.

5. Add 100 μ 1 supernatent from the hybrodoma culture to be tested to the well. Incubate for 24 hours at room temperature (incl. 0,05% S.A.).

6. Wash 2 times with washing buffer.

7. Wash 2 times with washing buffer - (without any S.A.).

8. Add 100 μl of interferon solution (f.x. as native interferons) containing 0,1 unit in total (wide the section concerning interfetitration) for 2 hours at room temperature.

9. Transfer the mixture (100 μl) to MDBK-cells, in microwells, which in advance have been grown to confluency using the same micro-trays as used for the interferon titrations (NUNC-Denmark) and incubate for 20 hours at 37°C (5% $CO_2$).

10. Add VSV at a predetermined concentration yielding a distinct CPE after 24 hours in control cultured cells not receiving any interferon.

11. Read the microtrays as usual (wide the section: interferon titration and references cited herein), taking into account that if the cells are destructed in one well the supernatent from the original hybridoma clone (culture) did contain antibodies which were able to bind (remove) the HuIFN-α: thus, this result signifies a positive clone.

## CLONING AND FURTHER GROWTH OF POSITIVE HYBRIDOMAS

Positive clones, identified as described above, were divided into several subclones by means of the "limited dilution technique" using conditioned medium and feeder layers (peritoneal macrophages) from Balb/c mice) such as described in details by Lovborg (1982). The subcloned hybridoma were all checked regularly for binding activity and if positive persistently the cells were allowed to grow further before injected (I.P.) into balb c mice which had received an immunostimulator in advance (f.x. Pristane, 4-6 days in advance). After 5-15 days (depending on the amount of injected cloned cells) new hybridoma cells of the correct specificity can be harvested together with large volumes of ascites fluids containing the desired antibody in an extreme high concentration (about more than 95% of all the immunoglobulins present are of the desired specificity). The hybridoma cells are separated from the ascites fluid subsequent to a centrifugation and can now, in turn, be used for another injection in new mice (Balb/c mice) whereby more ascites fluids can be obtained together with more hybridomas, and so forth, taking into account, that the hybridoma cells harvested from one mouse can be used for production of 20 new mice each yielding approximately the same number of hybridoma cells as the first. The hybridoma cells may also be grown in vitro in roller flasks using the relevant conditions such as already has been described in detail by Lovborg (1982) and in principle the same cells and antibodies will be obtained as described above using the mouse-system (in vivo).

## ISOLATION OF ANTI IFN- IMMUNOGLOBULINS

Since it is well known that the antibodies produced in ascites fluids are highly succeptible to proteolytic degradation from the proteolytic enzymes present in the ascites fluid the harvested ascites fluids were first cooled to +4°C and centrifuged (to remove hybridoma cells and other debris) before treated with ammonium sulphate whereby the immunoglobulins were presipitated. This isolation was performed immediately after harvesting the ascites fluids. The ammonium sulphate precip. immunoglobulins were kept at -20°C. Before titration a small aliquote was dissolved in f.x. 5 ml of medium, dialyzed versus 1 liter of PBS, before being checked in the semi-solid neutralization-test. The immunoglobulins were further purified by traditional methods such as gelfiltration, ionexchange chromatography, and the like.

CELLS

The mouse myeloma cell line used in the present invention is characterized as follows: Mouse Myeloma, line P3 x 63 NSI, HPRT-deficient, produces Kappa, non-immunoglobulin secreting. Medium contains 4.500 mg glucose pr. liter.

The above line was obtained from the Human Genetic Mutant Cell Repository (under cataloque nr.: GM 3573), Copewood and Davies Street, Camden, New Jersey, 08103, USA. (NIH Publication no. 81-20011, Revised Oct. 1981).

MEDIUM

The myeloma cells were grown in the same media composition as described by numerous other investigators, cf. Lovborg (1982), using RPMI 1640, 10% calf serum (new born), containing penicillin, streptomycin, gentamycin together with Napyruvate etc. as described by for example Lovborg (1982).

STORAGE OF HYBRIDOMA CELLS

The ascites fluids, which include the hybridoma cells of the desired specificity, are harvested from several mice (wide section: cloning and growing of cells) by centrifugation and washed 1 time with RPMI 1640 and fuged. (the remainder of the ascites fluid is processed as described elsewhere, wide the section: isolation of immunoglobulins). About $40 \times 10^6$ cells are resuspended in 5 ml of fresh Eagle's medium (incl. 20% calf serum) to which is added an equal part of 19-20% DMSC in RPMI 1640 (Dimethyl Sulfoxide, Merck & Co.) in a dropwise manner (10 drops pr. minn.) mixing the suspension thereby avoiding a local heat (which might be detrimental to the hybridoma cells). After mixing the said suspension, comprising the hybridoma cells, are divided into 1 ml ampules which are sealed and kept at $4°C$ for 24 hours, after which, they are transferred to a $N_2^-$ container whereby the ampules are cooled $1°C$ pr min (according to the instructions from the manufacturer producing the so-called "Linde-container"). The cells in the ampules are finally stored in liquid nitrogen. cf. Lovborg (1982).

THAWING OF THE CELLS

One ampule is thawed by immersion into a $37°C$ warm water bath. After centrifugation the cells are resuspended in new medium and counted and adjusted to contain $10^4$-$10^5$ cells pr ml. The medium is changed after 24 hours or possibly earlier, in case the cells already have adhered. The further growing is detailed described by Lovborg (1982). Alternatively, the thawn cells (from the ampoule) is directly injected into a Balb /c mouse (I.P.) whereby new cells and ascites fluid (containing the desired antibody) can be harvested in 3-7 days (depending on the amounts of cells injected).

MYCOPLASMA TEST

Primary human amnion cells (obtained from the department of gynaecology) are seeded on small cover slips (of glass) and after attachment the cover slips are ready for use (the cells should not reach each other as confluency tends to lower the sensitivity). The sample, consisting of the myeloma cells, is added to the surface of the cover slip (about 10-15 drops of the sample) by means of a Pasteur pipette and the cover slip (containing the sample) is carefully wrapped into a plastic film to avoid evaporation. If the original sample, consisting of myeloma cells, were infected by mycoplasma, the same mycoplasma would also infect the amnion cells. Normally, the latter are free from mycoplasma infections when taken fresh. (cf. later -control cultures). On the following day the cultures are stained by means of "Hoechst Stain" for 2 hours, according to the staining procedure given by the manufacturer (Hoechst, W. Germany): 1-2 grains of the stain is dissolved into 5 ml buffer at pH 5,5. Only a few drops should be used in the actual staining since the pH(5,5) is below physiological range (7,2). The stain is incubated for 2 hours with the cells, after which, the cells are fixed with methanol and dried. If the amnion cells have been infected with the mycoplasma (stemming from the original sample) small fluorescencing particles will clearly be seen in a fluorescencemicroscope as an indication for mycoplasma infection. If no fluorescing particles are found no infection has occurred. A control culture (i.e.: only amnion cells) is also included. (the latter should be negative). When the myeloma cells were found to contain mycoplasmas they were discarded and fresh, mycoplasma free, myeloma cells were taken from the cell freezer (liquid nitrogen container).

EXAMPLE 1.

One female Balb/c mouse was immunized as described in the section. Immunization (wide also the section: Preparation of immunogens) using 100.000 units of HuIFN-$\alpha$ (stabilized with SDS) incl. F.A. followed by the usual weekly shots comprising 30-40.000 units (I.P.) and finally, three days before fusion, the mouse received a booster of 100.00 units as previously described. During the period of immunization the "traditional" neutralization titer (wide the section: Neutralization) were obtained:

TABLE 1

| NEUTRALIZATION TITERS OBSERVED IN THE IMMUNIZED MOUSE | |
|---|---|
| Week | neutralization units/ml serum |
| 1-3 | 0 |
| 3-5 | 50-80 |
| 7 | 1500 |

After the fusion had been performed by means of the spleen cells from the immunized animal and the NSI cells (wide section: Cells) the presence of 62 living clones was established by microscopic inspections (in Costar Trays, NUNC, Denmark. Cf. Lovborg (1982)). All supernatants (62) were checked by the semi-solid neutralization method (wide the said section) and only two clones were found to be positive (partially). The two clones were further grown and subcloned and during this procedure (wide section: Cloning and further growth of positive hybridomas) one positive clone was lost due to a mistake during the cloning procedure, the other (LO-22) was further propagated in Balb/c mice, as previously described by yielding 5,5 ml of ascites fluid together with a large amount of hybridoma cells (after four weeks subsequent to the injection (I.P.) of the primally, subcloned hybridoma cells estimated to 1000 in total. After isolation the suspended cells were further injected into 10 new Balb/c mice as previously described, and after 7 days 65 ml of ascites fluid was harvested together with a large number of hybridoma cells. Some of these were frozen, serving as a stock culture, others were transplanted into new mice. All the cell-free ascites fluids were precipitated and isolated as described in the section: Isolation of anti-HuIFN-$\alpha$ immunoglobulins. (several grams were isolated at this stage).

Semi-solid neutralization assays were simultaneously performed during the above experiments. The first two positive clones, however, did only neutralize the native human leukocyte interferon (from virus induced buffy coats) partially, although the supernatants from the primary hybridoma cultures could be diluted more than 100-fold without reaching an end-point. The same tendency, at a much higher level, was seen when the LO-22 was grown as an ascites tumor: at a dilution of more than $1_0{}^7$ there was still a partial neutralization/binding observed. (wide the semi-solid neutralization method). At the early stage of these experiments, the above mentioned binding results obtained in the semi-solid binding assay lead to the impression that the LO-22 could only recognize part of the species of HuIFN-$\alpha$, which was in line with the results from other groups (cf. Secher et al. (1980), Allan et al. (1980), Berg (1982)) and which was generally accepted as the scientific concensus.

In order to obtain more detailed and precise information about the binding capabilities of the LO-22 hybridoma antibody it was decided to carry out an antibody affinity chromatography taking into account that only half or even less of the applied leukocyte interferon would be expected to bind (cf. the partial neutralization observed on p. 17).

The immunoglobulins from 3 ml of ascites fluid from the original mouse (which had received only about 1000 cells I.P.) was isolated (wide the section: Isolation of anti IFN-$\alpha$ immunoglobulins) and coupled to CNBr-Activated Sepharose (cf. Berg (1977), Berg et al. (1980)) as previously described. The equilibrated column was loaded with 90 ml of crude, native human leukocyte interferon and after a thorough wash the column was eluted by means of low pH. The following, surprising and unexpected results were obtained, wide table 2: 96% of the interferon was removed from the original interferon preparation (input). Based on the results obtained in the semi-solid neutralization tests (wide table 1) the almost opposite result would have been anticipated (i.e.: only a portion was expected to be caught by the LO-22 column (f.x. 30%)).

TABLE 2

| ANTIBODY AFFINITY CHROMATOGRAPHY WITH LO-22 COLUMN | | | | |
|---|---|---|---|---|
| | volume ml | IFN-units human syst. | IFN-units bovine syst. | recovery % |
| Input | 90 | $4,1 \times 10^6$ | $4,8 \times 10^6$ | 100 |
| Wash | 100 | $2 \times 10^5$ | $9,5 \times 10^4$ | 4 |
| Eluate | 16 | $2,8 \times 10^6$ | $6,5 \times 10^6$ | 70 |

In order, further to analyze the binding properties of the LO-22 column, the wash (consisting of $2,0 \times 10^5$ human interferon units, cf. table 2) was concentrated to about 4 ml (by means of sucrose and dialysis tubing, cf. Berg et al (1980)) before being loaded back on the LO-22 column. The second wash was titrated in both human and bovine systems and the following results were obtained: less than 100 units (bovine) were detected, whereas, about 50.000 units (human) were detected. Furthermore, the second wash (consisting of about 50.000 human units) was able to neutralize anti HuIFN-$\beta$ completely by means of the traditional neutralization test (wide the section neutralization). The purified eluate from table 2 (consisting of $2,8 \times 10^6$ units (human) was not able to neutralize the said anti-HuIFN-$\beta$ in an analogous neutralization test, at all. Thus, the 50.000 units (human) seen in the second wash consists of another species - namely HuIFN-$\beta$. The above results are in complete agreement with for example Vilcek et al. (1977) and Berg (1982).

The eluate from LO-22 column (table 2) was analyzed on analytical thin slab SDS-PAGE (using gradient gels, 12%-24%, 25 cm long, cf. Berg. et al. (1980), (1982a), (1982b)) and an identical pattern of HuIFN-$\alpha$ species were seen when comparing with the previously published reports. Thus all species of HuIFN-$\alpha$ were seen (no "bovine" specie was present in the particular batch used in this experiment, cf. Berg et al (1982a), (1982b)). The SDS-PAGE was stained and the individual bands were cut out and titrated and eluted, as previously described by Berg et al. (1980), (1982a), (1982b), and the following results were obtained (table 3):

TABLE 3

| BIOLOGICAL PROFILE OF AN LO-22 ELUATE OBTAINED BY SDS-PAGE | | |
|---|---|---|
| Slice No. | interferon units (WISH cells) human units | interferon units (BDVK cells) bovine units |
| 1 | 0 | 0 |
| 2 | 15.000 | 140.000 |
| 3 | 20.000 | 45.000 |
| 4 | 5.000 | 15.000 |
| 5 | 5.000 | 15.000 |
| 6 | 15.000 | 45.000 |
| 7 | 3.000 | 15.000 |
| 8 | 1.300 | 7.000 |
| 9 | 500 | 4.000 |
| 10 | 15.000 | 13.000 |
| 11 | 300 | 5.000 |
| 12 | 300 | 13.000 |
| 13 | 7.000 | 15.000 |

All 12 species were found to be located in the usual molecular weight range of 16.000 -25.000 which is in complete agreement with previously described reports concerning the location and numbers of species present in a HuIFN-$\alpha$ preparation (Berg et al. (1982a), (1982b)). Probably, the LO-22 column is also able to recognize not previously detected species of HuIFN-$\alpha$ with a higher molecular weight since fractions containing interferon activity were also found around 30.000 (fraction 14; 15; and 16 yielding 600 human units/1000 bovine units; 1400/1800, and 60/180). These fractions are presently being more closely investigated.

The above results (from the antibody affinity chromatography) are, somehow, in contradiction to the results obtained by the semi-solid neutralization method (table 1) indicating only a partial binding of the species of HuIFN-$\alpha$ to LO-22 immunoglobulin. At present, the reason for this contradiction is thought to be based on the quality of the rabbit antimouse immunoglobulin used in the coating of the immunoplates (wide the section: semi-solid neturalization method).

The immunoglobulins produced from the LO-22 hybridoma cells were characterized as belonging to the IgG-class by means of Ouchterloni-tests in which several fractions of the ascites fluids (and supernatants from cultured LO-22 cells) were checked against rabbit antimouse immunoglobulins (DAKO-PATT - Denmark; Behring Werke -W. Germany) or goat antimouse IgG F(ab)$_2$ (Cappel) or goat antiIgM (heavy chain) (Cappel). Several controls were included (the NSI-cells alone yielded, as expected, no positive reaction against for example IgG). A heavy precipitate was seen especially between the hybridoma antibodies, LO-22, and goat antimouse IgG F(ab)$_2$ antiserum (Cappel).

COMMENTS TO EXAMPLE 1.

The above experiments which are detailed described have proved the following:

1. That the LO-22 hybridoma antibodies/column recognizes (bind) all species of HuIFN-$\alpha$ having the specific characterizations belonging to the HuIFN-$\alpha$ system. (cf. Berg (1982), Berg et al. (1980), (1982a), (1982b)).

2. That Human fibroblast interferon, HuIFN-$\beta$, which is known to be present in small amounts in a crude human leukocyte interferon preparation (about 1-2% of the total interferon activity has been shown to belong the HuIFN-$\beta$ specie - cf. Bert et al. (1975)) is not recognized by the LO-22 hybridoma antibodies/column.

INDUSTRIAL APPLICABILITY

The monoclonal antibodies/hybridoma (and the corresponding hybridoma cells including derived hybrid cells) which are able to recognize protein (or part(s) thereof) belonging antigenically to the Human Leukocyte Interferon System (HuIFN-$\alpha$) can be used as "universal" antibodies for purification and characterization of all the above mentioned proteins belonging to the HuIFN-$\alpha$ system.

REFERENCES.

Allen,G. & Fantes,K.H. (1980): A family of structural genes for human lymphoblastoid(leukocyte-type) interferon. Nature, 287, 408-411

Berg,K,,Ogburn,C.A.,Paucker,K.,Mogensen,K.E.,Cantell,K.(1975): Affinity chromatography of human leukocyte and diploid cell interferons on sepharose-bound antibodies. J.Immunol.,114,640-644.

Berg,K.,(1977): Sequential antibody affinity chromatography of human leukocyte interferon. Scand. J. Immunol.6,77-86.

Berg,K. & Heron,I.(1980): The complete purification of human leukocyte interferon. Scan.J.Immunol.,11, 489-502.

Berg,K.(1962) : Purification and Characterization of Murine and Human Interferons. A Review of the Literature of the 1970s. Acta. Pathol,Microbiol.,et Immunol.Scand.,Section C,Suppl. no. 279. pp.1-136.

Berg,K.& Heron,I. (1982a): Human leukocyte interferon comprizes a continuum of 13 interferon species.in:Lymohokines, (eds. Khan & Hill),Academic press,N.Y.,p.397-416.

Berg,K.,Secher,D. % Heron,I.(1982b): Purification and characterization of the HuIFN-$\alpha$ species. in: The Interferon System, A Review to 1982 - Part I. (Eds. Baron,Dianzani,Stanton), Texas Reports on Biology and Medicin,Vol. 41,Tx.,p.225-234.

Kennett,R.H.,McKern,T.J.,Bechtol,K.B.(1980): Monoclonal Antibodies, Hybridomas: A New Dimension in Biological Analyses,incl. appendix. Plenum press,N.Y.pp 1-421 .

Køhler et al.(1975): Nature,256,495-497.

Køhler et al.(1976): Eur. J. Immunol.,6,511-519.

- Part I. (Eds. Baron, Dianzani, Stanton), Texas Reports on Biology and Medicine, Vol. 41, Tx., p.225-234.

Kennett, R.H., McKern, T.J., Bechtol, K.B. (1980): Monoclonal Antibodies, Hybridomas: A New Dimension in Biological Anaylses, incl. appendix. Phenum press, N.Y. pp. 1-421.

Kohler et al. (1975): Nature, 256, 495-497.

Kohler et al. (1976): Eur. J Immunol., 6, 511-519.

Lovborg, U. (1982): Production and Maintenance, Trowbridge, London pp.1-65.

Rubinstein, M. (1982): Pure species of HuIFN-$\alpha$. Phil. Trans. R. Soc. Lond. B. 299.

Secher, D. & Burke, D.C. (1980): A monoclonal antibody for large-scale purification of human leukocyte interferon. Nature, 285, 446-450.

EP 0 139 676 B1

Vilcek, J., Havell, E.A., Yamazaki, S. (1977): Antigenic, phisicochemical and biological characterization of human inteferons. Ann. N.Y. Acad. Sciences, 284, 703-710.
Vilcek, J, Gresser, I., Merigan, T.C. (1980): Regulatory Functions of Interferons. Annals of New York Academy of Sciences, volume 350, pp.1-641.

**Claims**

1. A continuous cell line comprising hybridoma cells capable of producing monoclonal antibodies specific for at least one antigenic determinant common to the 12 species of Human Interferon-alpha demonstrating activity in the human system, and having molecular weights of at least 16 000 in SDS-PAGE, a species having a molecular weight of 16 600 being excluded, as described by Berg et al. in Human Lymphokines, Academic Press, N.Y. 1982, pages 397-416, obtainable by
   a) immunizing an animal with a primary immunogen comprising Human Interferon-alpha proteins admixed with an effective amount of sodium dodecyl sulfate to denature said proteins; and
   b) thereafter removing at least one antibody-producing cell from said animal and fusing genetic material from said antibody-producing cell with an immortal cell to form said hybridoma cell.

2. A monoclonal antibody specific for at least one antigenic determinant common to the 12 species of Human Interferonalpha demonstrating activity in the human system, and having molecular weights of at least 16 000 in SDS-PAGE, a species having a molecular weight of 16 600 being excluded, as described by Berg et al. in Human Lymphokines, Academic Press, N.Y. 1982, pages 397-416.

3. A process for producing a hybridoma cell capable of producing monoclonal antibodies specific for at least one antigenic determinant common to the 12 species of Human Interferon-alpha demonstrating activity in the human system, and having molecular weights of at least 16 000 in SDS-PAGE, a species having a molecular weight of 16 600 being excluded, as described by Berg et al. in Human Lymphokines, Academic Press, N.Y. 1982, pages 397-416, comprising the steps of
   a) immunizing an animal with a primary immunogen comprising Human Interferon-alpha proteins admixed with an effective amount of sodium dodecyl sulfate to denature said proteins; and
   b) thereafter removing at least one antibody-producing cell from said animal and fusing genetic material from said antibody-producing cell with an immortal cell to form said hybridoma cell.

4. A process according to Claim 3 wherein said effective amount of sodium dodecyl sulfate is between about 0.01% to about 5% by volume of immunogen.

5. Use of the hybridoma cells of Claim 1 for detecting Human Interferon-alpha in a human body fluid, comprising the steps of:
   a) causing said hybridoma cells to produce said monoclonal antibodies;
   b) contacting said monoclonal antibodies with said human body fluid suspected of containing said Human Interferon-alpha; and
   c) detecting whether binding occurs between said monoclonal antibodies and said human body fluid.

6. Use of the monoclonal antibodies of Claim 2 for detecting Human Interferon-alpha in a human body fluid.

**Patentansprüche**

1. Kontinuierliche Zelllinie, die Hybridomazellen umfaßt, die in der Lage sind, monoklonale Antikörper zu produzieren, die für mindestens eine antigene Determinante spezifisch sind, die den 12 humanen α-Interferon-Spezies gemeinsam sind, die Aktivität im Humansystem zeigen und die Molekulargewichte von mindestens 16 000 im SDS-PAGE besitzen, wobei eine Spezies mit einem Molekulargewicht von 16 600 ausgenommen ist, die von Berg et al. in Human Lymphokines, Academic Press, N. Y. 1982, S. 397 - 416 beschrieben ist, erhältlich durch:
   a) Immunisieren eines Tieres mit einem primären Immunogen, das humane α-Interferon-Proteine, vermischt mit einer effektiven Menge Natriumdodecylsulfat, um diese Proteine zu denaturieren, umfaßt und
   b) nachfolgende Entnahme von mindestens einer antikörperproduzierenden Zelle aus diesem Tier und Fusionieren des genetischen Materials aus dieser Antikörper produzierenden Zelle mit einer unsterblichen Zelle, um die Hybridomazelle zu bilden.

2. Monoklonaler Antikörper, der für mindestens eine der antigenen Determinanten spezifisch ist, die den 12 humanen α-Interferon-Speziesgemeinsam sind, die Aktivität im Humansystem zeigen und die Molekulargewichte von mindestens 16 000 im SDS-PAGE besitzen, wobei eine Spezies mit einem Molekulargewicht von 16 600 ausgenommen ist, die von Berg et al. in Human Lymphokines, Academic Press, N. Y. 1982, S. 397 - 416 beschrieben ist.

3. Verfahren zur Herstellung einer Hybridomazelle, die in der Lage ist, monoklonale Antikörper zu bilden, die für mindestens eine der antigenen Determinanten spezifisch sind, die den 12 humanen α-Interferon-Spezies gemeinsam sind, die Aktivität im Humansystem zeigen und die Molekulargewichte von mindestens 16 000 im SDS-PAGE besitzen, wobei eine Spezies mit einem Molekulargewicht von 16 600 ausgenommen ist, die von Berg et al. in Human Lymphokines, Academic Press, N. Y. 1982, S. 397 - 416 beschrieben ist, welches die Schritte

a) Immunisieren eines Tieres mit einem primären Immunogen, das humane α-Interferon-Proteine, vermischt mit einer effektiven Menge Natriumdodecylsulfat, um diese Proteine zu denaturieren, umfaßt und

b) nachfolgende Entnahme von mindestens einer antikörperproduzierenden Zelle aus diesem Tier und Fusionieren des genetischen Materials aus dieser Antikörper produzierenden Zelle mit einer unsterblichen Zelle, um die Hybridomazelle zu bilden, umfaßt.

4. Verfahren nach Anspruch 3, bei dem die effektive Menge Natriumdodecylsulfat zwischen etwa 0,01 Vol.% und etwa 5 Vol.% des Immunogens beträgt.

5. Verwendung der Hybridomazellen nach Anspruch 1 zum Nachweisen von humanem α-Interferon in einer menschlichen Körperflüssigkeit, welche die Schritte umfaßt:

a) Anregen der Hybridomazellen zur Produktion der monoklonalen Antikörper;

b) Kontaktieren der monoklonalen Antikörper mit der menschlichen Körperflüssigkeit, von der angenommen wird, daß sie das humane α-Interferon enthält; und

c) Bestimmen, ob eine Bindung zwischen den monoklonalen Antikörpern und der menschlichen Körperflüssigkeit stattfindet,

6. Verwendung der monoklonalen Antikörper nach Anspruch 2 zum Nachweisen von humanem α-Interferon in einer menschlichen Körperflüssigkeit.

**Revendications**

1. Lignée cellulaire continue comprenant des hybridomes capable de produire des anticorps monoclonaux spécifiques d'au moins un déterminant antigènique commun aux douzes espèces d'Interféron-alpha humain montrant une activité dans l'organisme humain et possédant un poids moléculaire d'au moins 16 000 en SDS-PAGE, à l'exclusion d'une espèce dont le poids moléculaire est de 16 600, décrite par Berg et al. dans Humain Lymphokines, Acad Press, N.Y. 1982, page 397-416, qui peut être obtenue:

a) en immunisant un animal à l'aide d'un immunogène primaire contenant des protéines d'Interféron-alpha humain ajoutées à une quantité de dodécylsulfate de sodium efficace pour dénaturer lesdites protéines et

b) en isolant ensuite au moins une cellule productrice d'anticorps à partir dudit animal et en fusionnant le matériel génétique de ladite cellule productrice d'anticorps avec une cellule immortelle afin de former ledit hybridome.

2. Anticorps monoclonal spécifique d'au moins un déterminant antigènique commun aux 12 espèces d'Interféron-alpha humain montrant une activité dans l'organisme humain et possédant un poids moléculaire d'au moins 16 000 en SDS-PAGE, à l'exclusion d'une espèce dont le poids moléculaire est de 16600, décrite par Berg et al. dans Humain Lymphokines, Acad Press, N.Y. 1982, page 397-416.

3. Procédé de production d'un hybridome capable de produire un anticorps monoclonal spécifique d'au moins un déterminant antigènique commun aux 12 espèces d'Interférons-alpha humains montrant une activité dans l'organisme humain et possédant un poids moléculaire d'au moins 16 000 en SDS-PAGE, à l'exclusion d'une espèce dont le poids moléculaire est de 16 600, décrite par Berg et al. dans

EP 0 139 676 B1

Humain Lymphokines, Acad Press, N.Y. 1982, page 397-416,comprenant les étapes consistant à:

a) immuniser un animal avec un immunogène primaire contenant des protéines d'Interféron-alpha humain ajoutées à une quantité de dodécylsulfate de sodium efficace pour dénaturer lesdites protéines et

b) isoler ensuite au moins une cellule productrice d'anticorps à partir dudit animal et fusionner le matériel génétique de ladite cellule productrice d'anticorps avec une cellule immortelle afin de former ledit hybridome.

4. Procédé selon la revendication 3 dans lequel ladite quantité efficace de dodécylsulfate de sodium est comprise entre 0,01% environ et 5% environ par volume d'immunogène.

5. Utilisation des hybridomes de la revendication 1 pour détecter l'Interféron-alpha humain dans un liquide du corps humain, comprenant les étapes consistant à :

a) induire la production dudit anticorps monoclonal par lesdits hybridomes,

b) mettre en contact ledit anticorps monoclonal et ledit liquide du corps humain soupçonné de contenir ledit interféron-alpha humain et

c) détecter s'il se produit une liaison entre ledit anticorps monoclonal et ledit liquide du corps humain.

6. Utilisation de l'anticorps monoclonal de la revendication 2 pour détecter l'interféron-alpha humain dans un liquide du corps humain.

13